(19) Europäisches Patentamt
European Patent Office
Office européen des brevets

(11) Veröffentlichungsnummer : **0 028 362**
**B1**

(12) # EUROPÄISCHE PATENTSCHRIFT

(45) Veröffentlichungstag der Patentschrift : **16.11.83**

(51) Int. Cl.³ : **C 07 C 69/15**, C 07 C 67/54

(21) Anmeldenummer : **80106460.1**

(22) Anmeldetag : **23.10.80**

(54) **Verfahren zur Abtrennung von Wasser aus Gemischen mit Vinylacetat und Essigsäure.**

(30) Priorität : **31.10.79 DE 2943985**

(43) Veröffentlichungstag der Anmeldung : **13.05.81 Patentblatt 81/19**

(45) Bekanntmachung des Hinweises auf die Patenterteilung : **16.11.83 Patentblatt 83/46**

(84) Benannte Vertragsstaaten : **BE DE FR GB IT NL**

(56) Entgegenhaltungen :
**DE A 1 807 738**
**DE A 2 335 673**
**DE A 2 610 624**

(73) Patentinhaber : **HOECHST AKTIENGESELLSCHAFT**
**Postfach 80 03 20**
**D-6230 Frankfurt am Main 80 (DE)**

**WACKER-CHEMIE GMBH**
**Prinzregentenstrasse 22**
**D-8000 München 22 (DE).**

(72) Erfinder : **Roscher, Günter, Dr.**
**Altkönigstrasse 7**
**D-6233 Kelkheim (Taunus) (DE)**
Erfinder : **Schmidt, Karl-Heinz, Dr.**
**Taubenberg 86**
**D-6270 Idstein/Taunus (DE)**
Erfinder : **Langner, Horst**
**Staufenstrasse 32**
**D-6234 Hattersheim am Main (DE)**
Erfinder : **Neu, Hermann, Dr.**
**Wilhelmstrasse 14**
**D-6078 Neu-Isenburg (DE)**
Erfinder : **Lienerth, Aladar, Dr.**
**Am Steinbruch 6**
**D-6233 Kelkheim (Taunus) (DE)**
Erfinder : **Dempf, Dominik, Dr.**
**Schifferlehnerstrasse 1**
**D-8261 Mehring-öd (DE)**
Erfinder : **Kaiser, Klaus**
**Orffstrasse 6a**
**D-8263 Burghausen/Salzach (DE)**

EP 0 028 362 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Abtrennung von Wasser aus Gemischen mit Vinylacetat und Essigsäure

Die Herstellung von Vinylacetat durch Umsetzung von Ethylen mit Essigsäure und Sauerstoff oder sauerstoffhaltigen Gasen an Festbettkatalysatoren in der Gasphase ist bereits bekannt. Die Reaktion erfolgt im allgemeinen bei Drucken von 1 bis 25 bar und Temperaturen von 100 bis 250 °C. Geeignete Katalysatoren enthalten einen Edelmetallanteil und einen Aktivatoranteil. Der Edelmetallanteil besteht aus Palladium und/oder dessen Verbindungen ; zusätzlich können noch Gold oder dessen Verbindungen anwesend sein. Der Aktivatoranteil besteht aus Verbindungen von Elementen der 1. Hauptgruppe und/oder der 2. Hauptgruppe und/oder Cadmium. Diese aktiven Komponenten werden auf Träger in feiner Verteilung aufgebracht, wobei als Trägermaterial im allgemeinen Kieselsäure oder Aluminiumoxid verwendet wird.

Im allgemeinen liegt der Palladiumgehalt im Katalysator zwischen 0,5 und 5 Gew.-%.

Wird Gold bzw. eine seiner Verbindungen eingesetzt, so wird es in einem Anteil von 0.01 bis 4 Gew.-% zugegeben.

Jeder einzelne Aktivator wird im allgemeinen ebenfalls in einem Anteil von 0,01 bis 4 Gew.-% zugegeben. Bei allen drei Prozentangaben wird jeweils der Metallanteil der Komponente auf die Gesamtmasse des Trägerkatalysators bezogen. Bevorzugt sind folgende Katalysatoren :

Palladium/Alkali/Cadmium sowie Palladium/Gold/Alkali, wobei Palladium bzw. Gold als Metalle oder Verbindungen im fertigen Katalysator vorliegen können und wobei als Alkalielement Kalium bevorzugt ist (in Form eines Carboxylats).

Besonders bevorzugt sind die Katalysatoren Palladiumacetat/Kaliumacetat/Cadmiumacetat, sowie Palladiumacetat/Bariumacetoaurat/Kaliumacetat.

Aufgrund der Stöchiometrie wird pro Mol Vinylacetat bereits ein Mol Wasser gebildet :

$$CH_2 = CH_2 + 1/2\ O_2 + CH_3COOH \rightarrow CH_2 = CHOOC \cdot CH_3 + H_2O$$

Da ein Teil des umgesetzten Ethylens jedoch zu $CO_2$ und Wasser verbrennt :

$$CH_2 = CH_2 + 3\ O_2 \rightarrow 2\ CO_2 + 2\ H_2O$$

wird pro Mol Vinylacetat mehr als ein Mol Wasser gebildet ; im allgemeinen beträgt die gebildete Wassermenge gewichtsmäßig etwa ein Viertel der hergestellten Vinylacetatmenge.

Das zur Reaktion eingesetzte Gemisch enthält einen mehrfachen molaren Überschuß über die Stöchiometrie an Ethylen. Dementsprechend ist der Ethylenumsatz bei der Reaktion gering und das nicht umgesetzte Ethylen muß im Kreislauf zur Reaktion zurückgeführt werden. Die Abtrennung des gebildeten Vinylacetats aus dem Gasgemisch hinter dem Vinylacetatreaktor erfolgt in zwei oder mehr Stufen. Zunächst wird das den Reaktor verlassende heiße Reaktionsgemisch, das im wesentlichen aus nicht umgesetztem Ethylen, nicht umgesetzter Essigsäure, nicht umgesetztem Sauerstoff, Vinylacetat, Reaktionswasser, $CO_2$, sowie mit dem Sauerstoff und Ethylen eingebrachten Inerten (z. B. $N_2$ und Argon) besteht, abgekühlt. Dabei wird der größte Teil der Essigsäure, des Vinylacetats und des Wassers kondensiert. Das entstehende flüssige Gemisch wird im folgenden als « Kondensat » bezeichnet. Ein Teil des Vinylacetats (sowie der Essigsäure und des Wassers) verbleibt dabei entsprechend dem Partialdruck im nicht kondensierten Restgas das hauptsächlich aus Ethylen, $CO_2$ und Inerten besteht. Dieser Vinylacetatanteil wird in einer mit Essigsäure als Absorptionsflüssigkeit betriebenen Waschkolonne aus dem Restgas vor dessen Rückführung zur Reaktion entfernt. Die entstehende Lösung, im folgenden auch als « Sumpfablauf der Essigsäurewäsche » bezeichnet, wird nach dem Stand der Technik mit dem Kondensat zu dem sogenannten « Rohvinylacetat » vereinigt. Das Kondensat enthält etwa 15-30 Gew.-% Vinylacetat und 6-11 Gew.-% Wasser, der Rest sind Essigsäure und Spuren anderer Nebenprodukte (wie Ethylacetat, Ethylendiacetat, Acetaldehyd). Die Kondensatmenge ist etwa 1-3 mal so groß wie die Menge des Sumpfablaufs der Essigsäurewäsche. Dieser enthält etwa 15-30 Gew.-% Vinylacetat und 1-3 Gew.-% Wasser, ist also wasserärmer als das Kondensat ; der Rest ist im wesentlichen Essigsäure. Das aus dem Kondensat und dem Sumpfablauf der Essigsäurewäsche gebildete Rohvinylacetat enthält etwa 15-30 Gew.-% Vinylacetat, 5-8 Gew.-% Wasser, der Rest ist im wesentlichen Essigsäure. Das Rohvinylacetat wird destillativ aufgetrennt in reines Vinylacetat und Essigsäure, die als sogenannte Rückessigsäure wieder der Reaktion zugeführt wird.

Die destillative Aufarbeitung des Rohvinylacetats wird im allg. nach einem der beiden folgenden Verfahren durchgeführt : Bei der einen Variante (DE-OS 1 807 738, DE-OS 1 768 412) werden in einer ersten Kolonne das Vinylacetat und das Wasser über Kopf destilliert und als Sumpf Essigsäure abgezogen.

Aus dem sich in zwei Phasen trennenden Destillat wird die Wasserphase abgezogen. Ein Teil der Vinylacetatphase wird als Rücklauf verwendet und dient dabei gleichzeitig als Azeotropbildner für das über Kopf zu destillierende Wasser. Der verbleibende Teil der (wassergesättigten) Vinylacetatphase wird in einer zweiten Kolonne getrocknet, indem ein Teil als Azeotrop mit dem noch darin enthaltenen Wasser über Kopf destilliert wird. Der andere Teil gelangt in den wasserfreien Sumpf dieser zweiten Kolonne und wird in einer dritten Kolonne zu reinem Vinylacetat, das über Kopf destilliert, und Hochsiedern sowie

Polymeren, die als Sumpf abgezogen werden, aufgearbeitet.

Bei der zweiten Variante der Rohvinylacetat-Aufarbeitung (DE-PS 1 282 014 und DE-PS 1 668 063) wird in einer ersten Kolonne mit Vinylacetat als Azeotropbildner das Wasser über Kopf destilliert ; aus dem sich in zwei Phasen trennenden Destillat wird nur die Wasserphase abgezogen und die gesamte Vinylacetatphase als Rücklauf in die Kolonne zurückgeführt. In einer besonderen Ausführungsform enthält diese Kolonne noch einen Aufsatz zur Abtrennung von Leichtsiedern (z. B. Acetaldehyd), die niedriger sieden als Vinylacetat. In diesem Fall werden Vinylacetat und Wasser unterhalb dieses Aufsatzes entnommen und einem Phasentrenner zugeführt. Die Wasserphase wird wieder abgezogen und die Vinylacetatphase wieder als Rücklauf unterhalb der Entnahmestelle für Vinylacetat/Wasser in die Kolonne zurückgeführt. Am Kopf des Kolonnenaufsatzes können dann direkt die Leichtsieder abgezogen werden. Als Sumpf der Kolonne wird ein wasserfreies Vinylacetat/Essigsäure-Gemisch abgezogen, das in einer zweiten Kolonne getrennt wird in reines Vinylacetat (Kopfprodukt) und Essigsäure (Sumpfprodukt).

Bei beiden Aufarbeitungsvarianten erfordert die Abtrennung des Wassers 60-80 % der insegesamt für die Gewinnung von reinem Vinylacetat aufzuwendenden Energie. Jede Verbesserung bei der destillativen Wasserabtrennung bedeutet deshalb eine wesentliche Verfahrensverbesserung. Ein Maß für den Energieverbrauch bei der Entwässerung ist der Prozentgehalt an Wasser im Destillat der Kolonne für die Azeotropentwässerung. Eine hohe Wasserkonzentration in dem genannten Destillat bedeutet, daß für die azeotrope Wasserausschleusung weniger Vinylacetat mitverdampft und daher weniger Energie aufgewendet wurde als bei kleinerer Wasserkonzentration im Destillat.

Gegenstand der Erfindung ist nun ein Verfahren, durch das der Energieverbrauch für die Entwässerung des Rohvinylacetats wesentlich vermindert wird.

Das Verfahren zur Abtrennung von Wasser aus dem Gasgemisch, das bei der Herstellung von Vinylacetat durch Umsetzung von Ethylen mit Essigsäure und Sauerstoff an Palladium oder Palladiumverbindungen enthaltenden Katalysatoren in der Gasphase gebildet wird und das Hauptbestandteile Essigsäure, Vinylacetat, Wasser, Kohlendioxid und Ethylen enthält, wobei man

a) durch Abkühlung des aus der Reaktionszone austretenden Gasgemisches ein die Hauptmenge der Essigsäure, des Vinylacetats und des Wassers enthaltendes Kondensat gewinnt

b) aus dem in Schritt a) nicht kondensierten Gas durch Absorption mit Essigsäure eine das restliche Vinylacetat und das restliche Wasser enthaltende essigsaure Lösung gewinnt,

ist dadurch gekennzeichnet, daß man

c) das in Schritt a) erhaltene Kondensat und die in Schritt b) erhaltene Lösung getrennt voneinander derselben Destillationskolonne zuführt und das Wasser mit dem Vinylacetat als Azeotropbildner über Kopf destilliert, wobei die Einspeisungsstelle des Kondensats 4-30 Böden oberhalb der Einspeisungsstelle der Lösung liegt, und

d) aus dem sich in eine Wasserphase und eine Vinylacetatphase trennenden Destillat die Wasserphase abzieht.

Die Vinylacetatphase wird anschließend beispielsweise wie in einem der beiden oben erwähnten Destillationsverfahren zu reinem Vinylacetat weiterverarbeitet.

In Schritt a) wird das Gasgemisch i. allg. auf 15-50 °C, vorzugsweise auf 25-40 °C abgekühlt. Die Absorption des restlichen Vinylacetats und Wassers in Schritt b) erfolgt i. allg. in einer Waschkolonne mit Essigsäure als Absorptionsmittel. Die in Schritt c) benutzte Destillationskolonne hat i. allg. 50-80 Böden. Die Einspeisungsstellen von Kondensat und Lösung haben einen Abstand von 4-30, vorzugsweise 6-20 Böden. Die Einspeisungsstelle des Kondensats liegt dabei i. allg. mindestens 5 Böden unterhalb des Kolonnenkopfes und mindestens 30 Böden oberhalb des Kolonnensumpfes.

Der Wassergehalt im Destillat der Kolonne ist bei der erfindungsgemäßen getrennten Einspeisung des wasserreicheren Kondensats aus Schritt a) und der wasserärmeren Lösung aus Schritt b) wesentlich höher als bei der gemeinsamen Einspeisung nach dem Stand der Technik.

Das Verfahren wird durch die folgenden Beispiele erläutert.

Allg. Versuchsbeschreibung (vgl. Figur) :

In einer Glockenbodenkolonne (1) aus Glas (mit Vakuummantel, elektrischer Sumpfbeheizung, 60 Böden, Innendurchmesser 50 mm) wird (wasserreiches) Kondensat aus Vorratsegefäß (2) durch Leitung (3) über Pumpe (4) und Strömungsmesser (5) dem 45. Boden zugeführt. Aus Vorratsgefäß (6) wird (wasserarmer) Sumpfablauf der Essigsäurewäsche durch Leitung (7) über Pumpe (8) und Strömungsmesser (9) wahlweise entweder über Ventil (10) und Leitung (3) gemeinsam mit dem Kondensat dem 45. Boden der Kolonne (1) zugeführt, oder getrennt vom Kondensat durch Leitung (11) über Ventil (12) dem 30. Boden. Die am Kopf der Kolonne (1) anfallenden Dämpfe gelangen durch Leitung (13) in den Wasserkühler (14). Das dort verflüssigte Destillat gelangt über Leitung (15) (15) in das Sammelgefäß (16), wo es sich in zwei Phasen trennt. Die wäßrige untere Phase (17) wird über Leitung (18) abgezogen und verworfen. Die Vinylacetatphase (19) wird über Leitung (20) abgezogen. Sie wird gemäß der oben beschriebenen 2. Variante der Vinylacetataufarbeitung über Pumpe (21) und Strömungsmesser (22) — nach Ausschleusung einer geringen Teilmenge über Leitung (23) und Strömungsmesser (24) zur Entfernung von kleinen Mengen Acetaldehyd, die durch Vinylacetat-Hydrolyse gebildet werden — als Rücklauf zum Kopf der Kolonne (1) zurückgeführt. Aus Vorratsgefäß (25) wird durch Leitung (26) über

Pumpe (27), Strömungsmesser (28) und Leitung (20) eine Stabilisatorlösung auf den Kopf von Kolonne (1) gepumpt. Der Flüssigkeitsstand im Sumpf (29) der Kolonne (1) wird konstant gehalten, indem man über Leitung (30) und Strömungsmesser (31) eine entsprechende Menge wasserfreies Vinylacetat/Essigsäure-Gemisch abzieht.

## Vergleichsbeispiel

Man benutzt die eben beschriebene Versuchsanordnung. Vorratsgefäß (2) enthält eine Mischung von 30 Gew.-% Vinylacetat, 11 Gew.-% Wasser, 59 Gew.-% Essigsäure (= Kondensat, das durch Abkühlung des den Vinylacetatreaktor verlassenden Gasgemischs erhalten wurde). Vorratsgefäß (6) enthält eine Mischung von 30 Gew.-% Vinylacetat, 2 Gew.-% Wasser, 68 Gew.-% Essigsaüre (= Sumpfablauf aus der Essigsäurewäsche des nicht kondensierten Restgases).

Aus Vorratsgefäß (2) werden dem 45. Boden der Kolonne (1) 1 000 g/h Mischung zugeführt und aus Vorratsgefäß (6) bei geöffnetem Ventil (10) und geschlossenem Ventil (12) 600 g/h Mischung. Dies entspricht einem Gesamtgemischzulauf auf den 45. boden der Kolonne (1) von 1 600 g/h, der 30 Gew.-% Vinylacetat, 7.6 Gew.-% Wasser und 62.4 Gew.-% Essigsäure enthält. Die Sumpfbeheizung der Kolonne (1) wird nun so eingestellt, daß 2 400 g/h an organischer Phase in Destillatsammelgefäß (16) anfallen. Die gesamte organische Phase (19) wird über Pumpe (21) und Strömungsmesser (22) auf den Kopf der Kolonne (1) zurückgepumpt. Über Strömungsmesser (24) werden 20 g/h aus diesem Rücklauf ausgeschleust.

Aus Vorratsgefäß (25) wird eine Lösung von 1 Gew.-% Hydrochinon in Vinylacetat auf den Kopf der Kolonne (1) gepumpt, die Menge Beträgt 20 g/h.

Am Sumpf der Kolonne (1) werden 1 500 g/h Vinylacetat/Essigsäure-Gemisch mit 1.9 Gew.-% Wassergehalt abgezogen.

Im Destillatsammelgefäß (16) fallen 95 g/h wäßrige Phase (17) an, das sind 3,8 Gew.-% des gesamten Destillats (wäßrige plus organische Phase).

## Beispiel

Man geht vor wie im Vergleichsbeispiel, jedoch werden die beiden Mischungen in Kolonne (1) jetzt an verschiedenen Stellen eingespeist. Die Mischung aus Vorratsgefäß (2) wird zwar wie im Vergleichsbeispiel dem 45. Boden der Kolonne (1) zugeführt, die Zuführung der Mischung aus Vorratsgefäß (6) erfolgt jedoch jetzt bei geschlossenem Ventil (10) und geöffnetem Ventil (12) auf den 30. Boden der Kolonne (1). Am Sumpf der Kolonne (1) werden 1 480 g/h Vinylacetat/Essigsäure-Gemisch abgezogen, dessen Wassergehalt 0.3 Gew.-% beträgt.

Im Destillatsammelgefäß (16) fallen 2 400 g/h organische Phase (19) und 120 g/h wäßrige Phase (17) an, das heißt, 4.8 Gew.-% des gesamten Destillats entfallen auf die wäßrige Phase.

Gegenüber dem Vergleichsbeispiel ist die Wasserkonzentration (dh. der Gewichtsanteil der wäßrigen Phase) im Destillat von 3,8 Gew.-% auf 4,8 Gew.-% erhöht, das bedeutet eine relative Erhöhung um ca. 25 %.

Das bedeutet eine Einsparung an Destillationsenergie, bezogen auf die Gesamtdestillation (Verdampfungsenergie für organische plus Wasserphase) von ca. 20 % gegenüber dem Vergleichsbeispiel.

## Ansprüche

1. Verfahren zur Abtrennung von Wasser aus dem Gasgemisch, das bei der Herstellung von Vinylacetat durch umsetzung von Ethylen mit Essigsäure und Sauerstoff an Palladium oder Palladiumverbindungen enthaltenden Katalysatoren in der Gasphase gebildet wird und das als Hauptbestandteile Essigsäure, Vinylacetat, Wasser, Kohlendioxid und Ethylen enthält, wobei man

a) durch Abkühlung des aus der Reaktionszone austretenden Gasgemisches ein die Hauptmenge der Essigsäure, des Vinylacetats und des Wassers enthaltendes Kondensat gewinnt,

b) aus dem in Schritt a) nicht kondensierten Gas durch Absorption mit Essigsäure eine das restliche Vinylacetat und das restliche Wasser enthaltende essigsaure Lösung gewinnt,

dadurch gekennzeichnet, daß man

c) das in Schritt a) erhaltene Kondensat und die in Schritt b) erhaltene Lösung getrennt voneinander derselben Destillationskolonne zuführt und das Wasser mit dem Vinylacetat als Azeotropbildner über Kopf destilliert, wobei die Einspeisungsstelle des Kondensats 4-30 Böden oberhalb der Einspeisungsstelle der Lösung liegt, und

d) aus dem sich in eine Wasserphase und eine Vinylacetatphase trennenden Destillat die Wasserphase abzieht.

2. Verfahren nach Anspruch 1, dadurch gekennzeichnet, daß die in Schritt c) benutzte Destillationskolonne 50 bis 80 Böden hat.

3. Verfahren nach Anspruch 2, dadurch gekennzeichnet, daß die Einspeisungsstelle des Kondensats 6-20 Böden oberhalb der Einspeisungsstelle der Lösung liegt.

**Claims**

1. Process for separating water from the gas mixture, obtained in the manufacture of vinyl acetate by reacting ethylene with acetic acid and oxygen in the gaseous phase in contact with catalysts containing palladium or palladium compounds, which gas mixture consists essentially of acetic acid, vinyl acetate, water, carbon dioxide and ethylene, wherein

a) the gas mixture leaving the reaction zone is condensed to give a condensate containing the main amount of acetic acid, of vinyl acetate and of water and

b) an acetic acid solution containing the residual amounts of vinyl acetate and water is prepared by absorption of the gas non condensed in step a) in acetic acid, characterized by

c) introducing the condensate obtained in step a) and the solution obtained in step b) separately from each other into the same distillation column, the feeding point of the condensate being 4 to 30 trays above the feeding point of the solution, distilling off the water at the head of the column as an azeotrope together with the vinyl acetate and

d) removing the water phase from the distillate separating into a water phase and a vinyl acetate phase.

2. The process of claim 1, characterized in that the distillation column used in step c) has 50 to 80 trays.

3. The process of claim 2, characterized in that the feeding point of the condensate is 6 to 20 trays above the feeding point of the solution.

**Revendications**

1. Procédé pour séparer l'eau du mélange gazeux qui se forme lors de la préparation de l'acétate de vinyle par la réaction de l'éthylène avec l'acide acétique et l'oxygène, en phase gazeuse sur des catalyseurs contenant du palladium ou des composés du palladium, et qui contient en tant que constituants principaux de l'acide acétique, de l'acétate de vinyle, de l'eau, du dioxyde de carbone et de l'éthylène, à l'occasion duquel

a) on obtient, par refroidissement du mélange gazeux sortant de la zone de réaction, un condensat contenant la plus grande partie de l'acide acétique, de l'acétate de vinyle et de l'eau,

b) on obtient, à partir du gaz non condensé dans l'étape a), par absorption par l'acide acétique, une solution acétique contenant l'acétate de vinyle et l'eau résiduelle, caractérisé en ce que,

c) on envoie séparément l'un de l'autre, à la même colonne de distillation, le condensat obtenu dans l'étape a) et la solution obtenue dans l'étape b), et que l'on distille l'eau en tête, avec l'acétate de vinyle en tant que composant azéotropique, le point d'introduction du condensat se trouvant 4-30 plateaux au-dessus du point d'introduction de la solution, et

d) en ce qu'on extrait, du distillat qui se sépare en une phase aqueuse et une phase acétate de vinyle, la phase aqueuse.

2. Procédé selon la revendication 1, caractérisé en ce que la colonne de distillation utilisée dans l'étape c) a de 50 à 80 plateaux.

3. Procédé selon la revendication 2, caractérisé en ce que le point d'introduction du condensat se trouve 6 à 20 plateaux au-dessus du point d'introduction de la solution.

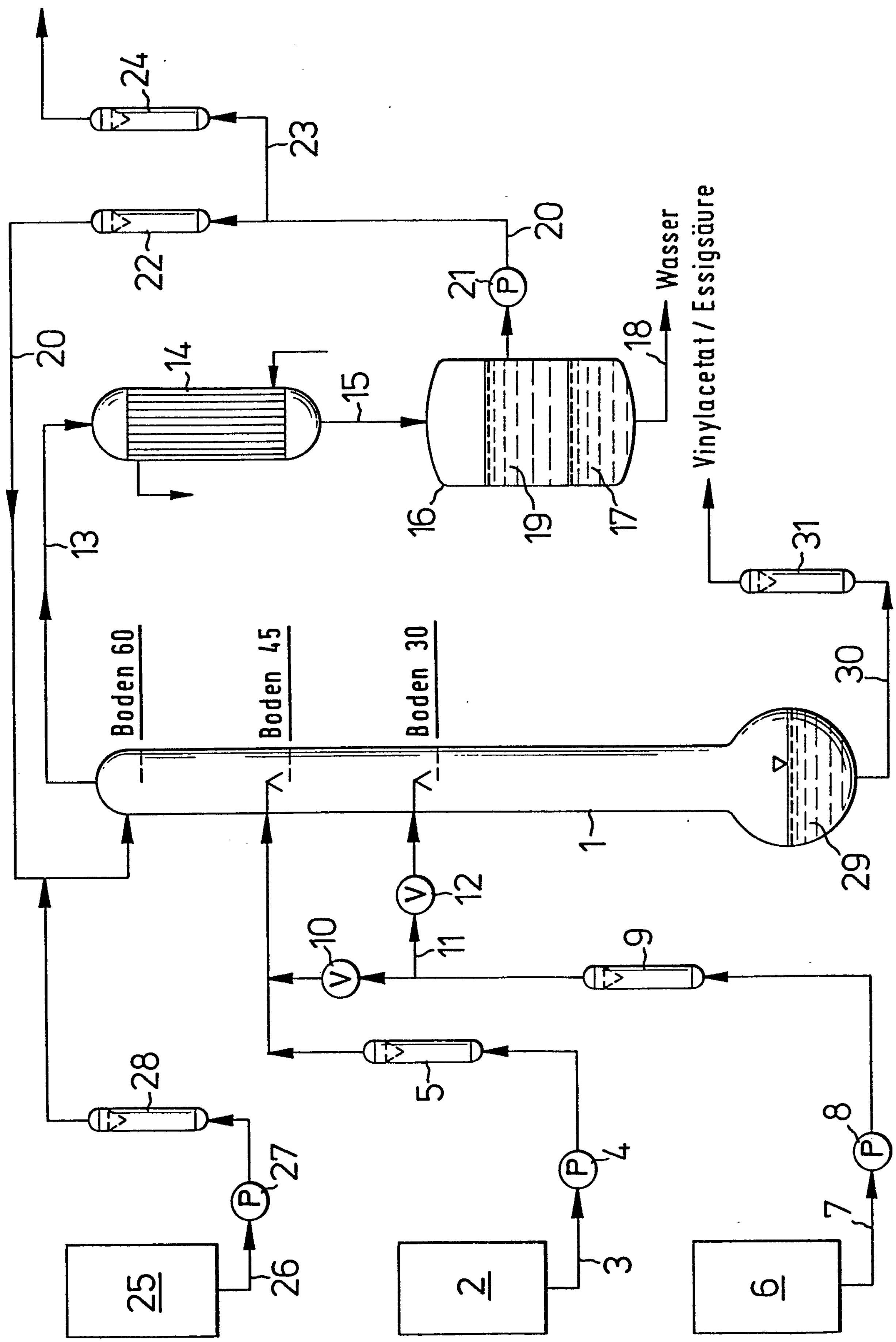
Boden 60
Boden 45
Boden 30
Wasser
Vinylacetat / Essigsäure
1
2
3
4
5
6
7
8
9
10
11
12
13
14
15
16
17
18
19
20
21
22
23
24
25
26
27
28
29
30
31